# FASCICULE DE BREVET EUROPEEN

(11) **EP 1 452 203 B1**
(45) Date de publication et mention de la délivrance du brevet: **29.07.2009**
(21) Numéro de dépôt: 04011635.2
(22) Date de dépôt: 17.12.1998
(51) Int. Cl.: A61N 1/30, A61F 9/00

(54) **Dispositif de iontophorese oculaire pour transfert de plusieurs produits actifs**
Vorrichtung zur okularen Iontophorese zur Verabreichung mehrerer Wirkstoffe
Ocular iontophoresis device for transferring a plurality of active ingredients

(30) Priorité: 05.01.1998 FR 9800009
(43) Date de publication de la demande: 01.09.2004
(62) Demande divisionnaire de: 98403189.8
(73) Titulaire: Eyegate Pharma SAS, 75012 Paris (FR)
(72) Inventeur: Parel, Jean-Marie, Miami Shores Floride 33138 (US); Behar, Francine, 75016 Paris (FR)
(74) Mandataire: Callon de Lamarck, Jean-Robert

(56) Documents cités:
- US-A- 2 525 381
- US-A- 4 564 016
- OLEG IVANOVICH LEBEDEV: "ELECTROPHORETIC TRIALS IN THE EARLY DIAGNOSIS OF PRIMARY GLAUCOMA" DISSERTATION IN COMPETITION FOR THE ACADEMIC DEGREE OF CANDIDATE OF THE MEDICAL SCIENCES, XX, XX, 1983, pages A,1-18,26, XP002932803
- KISELEN ET AL: "PROCEDURE FOR THE ADMINISTRATION OF DRUGS IN GELS TO OCULAR TISSUESTHROUGH THE USE OF ELECTROPHORESIS" PROCEDURE FOR THE ADMINISTRATION OF DRUGS IN GELS TO OCULAR TISSUES THROUGH THE USE OF ELECTROPHORESIS, XX, XX, 1984, pages 1-13,R01, XP002932802
- SALLMANN VON L: "IONTOPHORETIC INTRODUCTION OF ATROPINE AND SCOPOLAMINE INTO THE RABBIT EYE" ARCHIVES OF OPHTHALMOLOGY, XX, XX, vol. 290, 1943, pages 711-719, XP002932801 ISSN: 0003-9950
- SARRAF D ET AL: "The Role of iontophoresis in Ocular Drug Delivery" JOURNAL OF OCULAR PHARMACOLOGY, MARY ANN LIEBERT, INC. NEW YORK, NY, US, vol. 1, no. 10, 1994, pages 69-81, XP002077524 ISSN: 8756-3320
- BEHAR-COHEN F F ET AL: "IONTOPHORESIS OF DEXAMETHASONE IN THE TREATMENT OF ENDOTOXIN-INDUCED-UVEITIS IN RATS" EXPERIMENTAL EYE RESEARCH, ACADEMIC PRESS LTD., LONDON, GB, vol. 65, no. 65, 1997, pages 533-545, XP002932804 ISSN: 0014-4835

## Description

La présente invention a pour objet un dispositif de transfert intraoculaire de produits actifs par iontophorèse.

L'iontophorèse est une technique qui a été proposée dès 1747 par VERRATI et qui consiste en l'administration notamment de médicaments dans l'organisme à travers les tissus à l'aide d'un champ électrique mettant en jeu une faible différence de potentiel. L'électrode active, qui est en contact avec le médicament est disposée à l'endroit à traiter alors qu'une deuxième électrode, destinée à fermer le circuit électrique est placée à un autre endroit du corps.

Le champ électrique facilite la migration des produits actifs de préférence ionisés. Cette technique est couramment utilisée pour le traitement des maladies dermatologiques et il existe à cet effet différents dispositifs qui sont disponibles dans le commerce.

L'iontophorèse appliquée au traitement de l'oeil a fait l'objet de nombreuses expérimentations animales et de quelques tests cliniques, à l'aide de différents dispositifs.

Des dispositifs connus mettent en oeuvre un tampon imbibé d'une solution contenant un médicament, et qui est en contact avec la surface de la cornée et de la sclère. D'autres dispositifs mettent en oeuvre une cupule ou une pipette. Un dispositif mettant en oeuvre une cupule est par exemple décrit dans le Brevet des Etats-Unis US 4 564 016 (David M. MAURICE). Dans celui-ci, l'administration du médicament s'effectue de manière quasi-ponctuelle à travers la sclère.

D'une manière générale, les auteurs constatent une mauvaise reproductibilité de leurs résultats, qu'ils imputent soit à l'existence de différences entre les animaux testés, soit à des phénomènes biologiques inexpliqués. De surcroît, certaines techniques opératoires impliquent la mise en oeuvre d'une électrode active de très petite surface avec une densité de courant très élevée qui augmente le risque de dommages causés aux tissus, ces dommages pouvant aller jusqu'à la présence de brûlures. C'est le cas en particulier du dispositif décrit dans le Brevet US 4 564 016 précité qui préconise une densité de courant d'au moins 50 mA/cm² et pouvant même atteindre 2000 mA/cm².

Certaines expériences ont été réalisées avec des solutions alcalines dont le pH élevé a pour conséquence un endommagement tissulaire local. Par exemple, l'article de T.T. LAM et Collaborateurs "Intravitreal Delivery of Ganciclovir in Rabbits by Transscleral lontophoresis" publié dans le Journal of Ocular Pharmacology 10(3) p. 571-575 (1994), décrit l'administration ponctuelle d'une solution dont le pH de 10,8 n'est pas envisageable en dehors du laboratoire.

L'article de F. BEHAR-COHEN et Collaborateurs, intitulé "lontophoresis of Dexamethasone in the Treatment of Endotoxin-Induced-Uveitis in Rats" paru dans la Revue Experimental Eye Research, 1997-65 p. 533-545 (oct.1997), concerne une iontophorèse transcornéosclérale effectuée sur des rats, en vue du traitement de l'uvéite, c'est-à-dire une pathologie affectant l'uvée. Selon cette technique, la diffusion du médicament s'effectue essentiellement à travers la cornée, puis diffuse dans les milieux oculaires.

Un autre dispositif de l'état de la technique est divulgué par l'article de Kiselev et al. "Procedure for the administration of drugs in gels to ocular tissues through the use of electrophoresis", publié par le Ministry of health of the RSFSR, 1984.

En pratique, le peu de reproductibilité des résultats expérimentaux généralement obtenus et surtout la description de brûlures et de nécroses tissulaires sur le site d'application des dispositifs d'iontophorèse, a eu pour conséquence que l'iontophorèse trans-oculaire est restée au stade du laboratoire et n'est toujours pas reconnue en tant que méthode de traitement des patients.

L'invention a pour objet un dispositif de transfert d'au moins un produit actif dans le globe oculaire par iontophorèse, qui permette de réaliser des traitements ambulatoires de manière reproductible.

L'invention concerne ainsi un dispositif de transfert d'au moins un produit actif dans le globe oculaire humain par iontophorèse tel que revendiqué dans la revendication 1. Les régions du globe oculaire en regard de l'électrode sont le limbe cornéoscléral, la conjonctive et/ou la sclère et/ou le corps ciliaire et/ou la racine de l'iris et/ou la parsplana et/ou le vitré antérieur, et/ou la rétine non détachable non fonctionnelle.

Etant donné que le transfert s'effectue au travers d'un ou plusieurs tissus oculaires situés en périphérie de la cornée sur une large surface d'application, la reproductibilité, l'homogénéité de transfert et l'efficacité sont augmentées. Ces tissus s'imprègnent du médicament (ou produit actif) qui peut même s'y concentrer alors que les concentrations dans les milieux oculaires restent peu élevées. Ces concentrations ne reflètent pas les concentrations de médicament intratissulaires. Le médicament n'est ainsi pas éliminé rapidement par le renouvellement des liquides oculaires (humeur aqueuse AH et le vitré V).

D'autre part, étant donné que le produit actif n'est pas en contact avec la cornée, on évite les inconvénients de l'iontophorèse transcornéale et le risque de lésions endothéliales, à savoir l'existence après l'intervention de troubles de la vue liés, soit à des lésions endothéliales, soit à des lésions épithéliales transitoires, soit à des dépôts transitoires de produits actifs, qui se traduisent par une vision floue. De ce fait, le traitement est véritablement ambulatoire.

Enfin, le traitement s'effectuant sur une couronne périphérique à la cornée, une région centrale cylindrique du dispositif peut être entièrement dégagée et de ce fait le praticien peut contrôler visuellement le positionnement centré du dispositif au cours de l'iontophorèse.

Tous les tissus oculaires peuvent être traités : conjonctive, cornée, sclère, iris, cristallin, corps ciliaire, choroïde, rétine, nerf optique.

En fonction des paramètres choisis pour le courant (intensité du courant, durée du traitement), certains tissus pourront être plus spécifiquement ciblés.

Pour un adulte (diamètre nominal de la cornée: 12 mm), l'électrode annulaire ou les électrodes en forme de secteurs annulaires, réalisée(s) par exemple par électrodéposition, peut (peuvent) avoir un diamètre intérieur compris entre 12,5 mm et 14 mm et un diamètre extérieur compris entre 17 mm et 22 mm, ce qui correspond à une surface comprise entre environ 75 mm² et 250 mm², et de préférence entre 17 mm et 20 mm. Le diamètre maximal est choisi de manière à ne pas atteindre la rétine fonctionnelle. Pour un enfant dont l'oeil n'a pas atteint la taille adulte, il faut adapter les dimensions en proportion. En d'autres termes, et dans le cas général, le diamètre intérieur di de l'électrode annulaire ou des électrodes est supérieur au diamètre D de la cornée et inférieur ou égal à 1,2D, et le diamètre extérieur de l'électrode annulaire ou des électrodes est supérieur ou égal à 1,4D et inférieur ou égal à 1,8D, et de préférence inférieur ou égal à 1,7D.

Le générateur de courant peut être un générateur de courant constant de densité nominale inférieure à 10mA/cm², qui comporte un dispositif de commande permettant d'appliquer ledit courant constant pendant une durée comprise par exemple entre 30 secondes et 10 minutes et plus particulièrement entre 1 minute et 10 minutes.

La densité du courant constant est avantageusement réglable entre 0,1mA/cm² et 5mA/cm², par exemple entre 0,2mA/cm² et 5mA/cm² ou bien entre 0,8mA/cm² et 5mA/cm².

L'application du courant peut s'effectuer de manière progressive, par exemple pendant les premières secondes, ce qui évite les réactions musculaires réflexes du patient.

Le courant est avantageusement fourni sous une tension comprise entre 1,5V et 9V et de préférence entre 2V et 8V.

La concentration du produit actif peut être quelconque. Elle est en particulier inférieure ou égale à la concentration à saturation du produit actif dans l'eau. Elle est de préférence supérieure ou égale à une concentration de seuil à partir de laquelle se produit une accumulation dans certains tissus de l'oeil suivie d'un relargage vers d'autres tissus.

Le produit actif disposé dans le réservoir présente un pH qui peut être avantageusement compris entre 6 et 8 et de préférence entre 7 et 7,6. On remarquera que le produit actif n'étant pas en contact avec la cornée, le pH choisi peut être sensiblement plus élevé qu'indiqué ci-dessus, parce que la conjonctive et la sclère sont moins sensibles à la fois sensitivement et lésionnellement à des pH un peu acides ou basiques. La cornée doit rester transparente. Toute modification des conditions physiologiques risque d'altérer ses caractéristiques tissulaires, donc sa transparence. La conjonctive est une muqueuse, la sclère est un tissu conjonctif. Ce sont deux tissus très résistants et dont la fonction n'est pas, dans la région d'application du traitement, directement impliquée dans la transmission des photons vers la rétine. Ce sont des tissus de soutien.

Le dispositif présente de préférence un dispositif de pompage permettant d'assurer une circulation d'une solution de produit actif, par exemple une solution médicamenteuse, dans le réservoir. Ceci permet d'une part d'éliminer les bulles de gaz susceptibles de se former au cours de l'iontophorèse, et d'autre part de maintenir sensiblement constante la composition et le pH de la solution pendant la durée de traitement et donc d'en améliorer la reproductibilité.

Le dispositif de l'invention présente un réservoir annulaire présentant une pluralité de compartiments en forme de secteurs annulaires et des électrodes en forme de secteur annulaire, qui peuvent délimiter le fond des secteurs annulaires.

Un autre dispositif divulgué est constitué par une lentille cornéenne pourvue sur sa face interne d'une électrode surfacique et dans laquelle est disposé un gel contenant au moins un produit actif, ou qui est elle-même de structure spongieuse et contient le produit actif, (par exemple une matrice réticulée).

De préférence, le dispositif comporte sur une face externe une électrode passive qui vient en contact avec la paupière partiellement fermée du patient, laquelle maintient en place le dispositif pendant la durée du traitement. Ceci procure également l'avantage d'un contact électrique amélioré, car en milieu aqueux.

D'autres caractéristiques et avantages de l'invention apparaîtront mieux à la lecture de la description qui va suivre, donnée à titre d'exemple non limitatif en liaison avec les dessins ci-annexés dans lesquels :
- La figure 1 représente en coupe un exemple de dispositif décrit dans l'article précité de F. BEHAR-COHEN et al.
- les figures 2a à 2c représentent respectivement en coupe, en vue de dessus et en perspective un exemple de dispositif qui ne fait pas partie de l'invention
- les figures 3a, 3b et 3c représentent respectivement en coupe, en vue de dessus et en perspective un dispositif selon l'invention permettant l'administration de trois produits actifs, par exemple trois médicaments
- la figure 4 représente en coupe une variante du dispositif selon les figures 3a à 3c
- les figures 5a et 5b représentent un dispositif selon l'invention, en forme de ménisque destiné à l'administration de trois produits actifs, par exemple trois médicaments, sous forme de gel
- les figures 6a à 6c représentent respectivement en perspective, en coupe, et en coupe partielle un dispositif de type ménisque qui ne fait pas partie de l'invention
- la figure 7 représente un mode de réalisation préféré d'un dispositif destiné à l'administration de plusieurs produits actifs, par exemple des médicaments
- les figures 8a et 8b sont des résultats d'un test effectué sur des lapins, avec en ordonnée la concentration en µg/g de tissu sec et en µg/ml pour les milieux oculaires, et en abscisse le temps en heures
- la figure 9 représente un dispositif selon l'invention tel que mis en place sur un oeil à traiter
- et la figure 10 représente en vue de dessus une variante préférée de mise en oeuvre de l'invention.

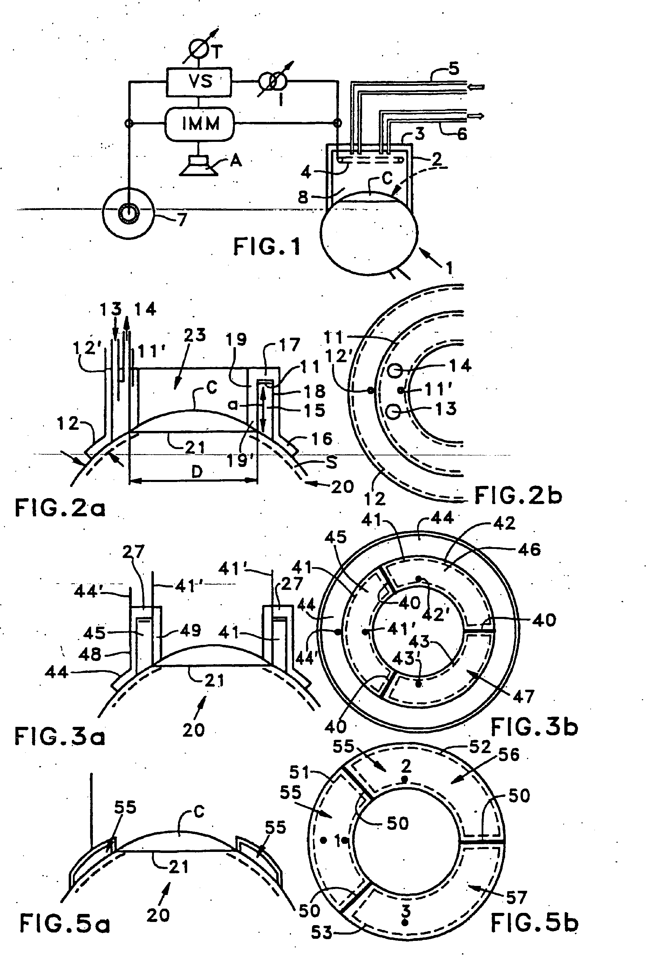

La figure 1 représente schématiquement le système d'iontophorèse mis en oeuvre dans le cadre de l'article précité de F. BEHAR-COHEN et Collaborateurs. Il comporte un réservoir 8 en polyméthylméthacrylate (PMMA) délimité par une paroi cylindrique 2 et un fond 3 à proximité duquel est disposée une électrode circulaire 4 en platine. Le réservoir 8 de diamètre 6 mm recouvre la cornée, le limbe et le premier millimètre de la sclère d'un rat. Un tube d'amenée 5 permet de remplir le réservoir 8 avec une solution dosée à raison d'1 mg de Dexaméthasone par ml d'une solution saline stérile de pH 7, et un tube d'évacuation 6 permet d'extraire les bulles d'air qui se forment au cours de l'iontophorèse. Une circulation continue de la solution permet de maintenir constant le pH de la solution en contact avec la cornée.

Une électrode de retour 7 est placée en contact avec une patte du rat.

Le système comporte également une source de tension VS, et un régulateur de courant I. Un dispositif IMM de mesure d'impédance permet de détecter toute discontinuité électrique et de déclencher une alarme A. La quantité de charges délivrée est affichée sur le générateur en fin de traitement et permet d'assurer la reproductibilité du traitement administré.

Les expériences ont été réalisées avec un courant de 400µA pendant 4 minutes, soit une densité de 1,2mA/cm² et une charge totale de 0,12 Coulombs soit 0,4C/cm².

Le dispositif selon l'invention permet un transfert de produit actif par exemple un médicament, essentiellement à travers au moins un tissu oculaire.

L'électrode active est avantageusement placée à une distance a de la surface de l'oeil du patient qui soit suffisante pour éviter un court-circuit, ou pour éviter qu'elle ne se trouve accidentellement en contact avec l'oeil. Cette distance a est de préférence au moins égale à 4 mm.

Le dispositif peut être réalisé en PMMA ou de préférence en silicone, par exemple du PDMS de dureté Shore 20, pour une meilleure étanchéité au niveau de l'oeil. Un autre matériau biocompatible utilisable est le polyuréthane, en particulier un polyuréthane hydrophile pour améliorer l'adhérence et l'élimination des bulles.

Le dispositif 10 présente une paroi annulaire 17 et deux parois latérales cylindriques intérieure 19 et extérieure 18 délimitant une région annulaire 15 formant un réservoir pour une solution active, par exemple médicamenteuse, à administrer par iontophorèse à la périphérie de la cornée C d'un oeil à traiter 20. L'extrémité de la paroi 18 adjacente à la paroi 17 repose par un rebord tronconique 16 sur la sclère S et l'extrémité de la paroi 19 adjacente à la paroi 17 repose par une zone tronconique 19' sur le pourtour de la cornée C de manière que seule une région périphérique à la cornée C et présentant un ou plusieurs tissus oculaires soit baignée par la solution médicamenteuse que contient le réservoir 15. Une électrode active annulaire 11 borde la paroi 17. Deux liaisons conductrices 11' et 12' permettent de connecter électriquement l'électrode active 11 et l'électrode de retour 12, qui est avantageusement disposée sur la face externe d'une couronne 16, de sorte que la paupière partiellement fermée du patient puisse venir en contact avec l'électrode 12 et fermer ainsi le circuit.

Alternativement, l'électrode de retour peut être séparée et disposée sur le front du patient au voisinage de l'oeil à traiter. Dans ce cas également, la paupière du patient peut reposer sur la couronne 16 pour maintenir en place le dispositif.

Des ouvertures 13 et 14 ménagées dans la paroi 17 permettent un remplissage du réservoir 15 et/ou une circulation de la solution médicamenteuse.

L'électrode annulaire plane 11 recouvre de préférence la totalité de la surface de la paroi 17 qui définit le fond du réservoir annulaire 15. Un recouvrement seulement partiel est certes envisageable, mais il ne peut influer que défavorablement sur l'efficacité du traitement. En tout état de cause, le réservoir 15 ne doit pas recouvrir de région de la cornée C.

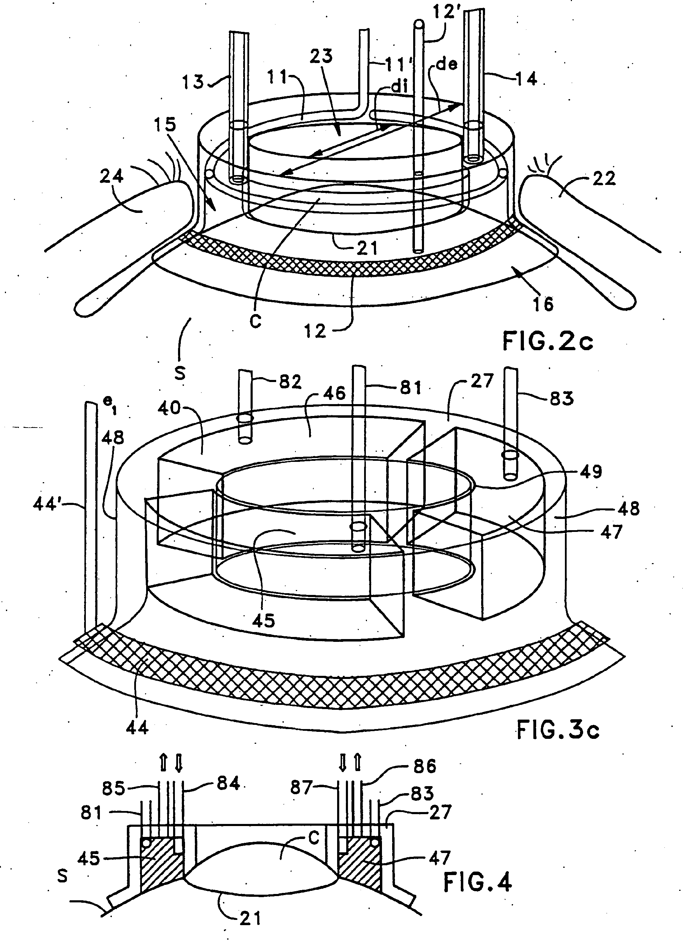

Le dispositif de l'invention représenté aux figures 3a, 3b et 3c permet l'administration de plusieurs produits actifs, par exemple des médicaments, ici 3, sous forme liquide ou de gel qui sont disposés chacun dans une de trois cavités en forme de secteur annulaire 45, 46 et 47 pourvue chacune d'une électrode active respectivement 41, 42 et 43. Le dispositif comporte une paroi annulaire 27, et deux parois cylindriques intérieure 49 et extérieure 48, et les secteurs sont délimités par des parois séparatrices 40. Il est posé sur l'oeil du patient de la même façon que le dispositif représenté aux figures 2a et 2b. Des liaisons conductrices 41', 42' et 43' traversent la paroi 27 pour alimenter électriquement les électrodes actives 41, 42 et 43.

Le dispositif représenté à la figure 4 se distingue par la présence de tubes de circulation de liquide qui sont présents pour chaque cavité 45, 46 et 47. Sur le dessin on voit les tubes 84, 85 et 86,87 correspondant aux cavités 45 et 46.

Le dispositif représenté aux figures 5a et 5b est un ménisque en forme de couronne. Il présente trois réservoirs 55, 56 et 57 dont chacun est destiné à recevoir un gel médicamenteux ou un matériau poreux, tel qu'une éponge, imprégné d'un produit actif par exemple un médicament. A chaque réservoir, est associée une électrode active respectivement 51, 52 et 53. Les réservoirs 55 en forme de secteurs sont délimités par des parois séparatrices 50.

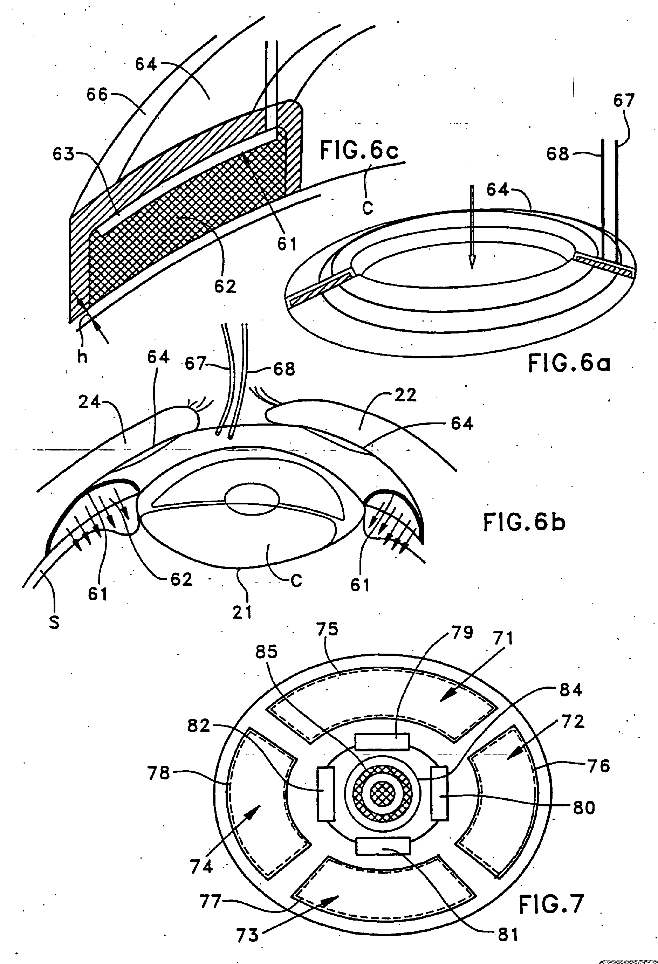

Le dispositif représenté aux figures 6a à 6c est un ménisque plat en forme de couronne réalisé en un matériau qui peut être celui d'une lentille cornéenne. L'espace central cylindrique 63 est dégagé et permet comme avec les autres modes de réalisation un contrôle visuel du positionnement centré du dispositif. Une électrode 61 par exemple formée par électrodéposition, recouvre la face interne légèrement concave 63 du fond de la cavité annulaire 62. Une électrode de retour 64, par exemple formée par électrodéposition, recouvre le pourtour de la face externe convexe 66 du fond de la cavité annulaire 62, de manière à permettre un contact électrique de retour par au moins une des paupières fermées 22, 24 du patient. Le passage des fils de contact électrique 67, 68 est disposé de manière à permettre leur sortie entre les paupières.

Le dispositif selon l'invention convient en général aux molécules simples ou aux assemblages moléculaires utilisés comme produit actif (par exemple des médicaments et/ou des peptides et/ou des protéines et/ou des fragments de gènes) et dont la masse moléculaire est inférieure à 100 kilodaltons.

On opère à courant continu, constant et régulé avec une densité de courant qui ne dépasse pas 10mA/cm². Cette densité de courant est avantageusement réglable entre 0,1mA/cm² et 5mA/cm², et par exemple entre 0,2mA/cm² et 5mA/cm². La fourchette de valeur préférée est comprise entre 0,8mA/cm² et 5mA/cm². La durée de traitement peut être comprise entre 30 secondes et 10 minutes. Elle peut en particulier être comprise entre 1 minute et 10 minutes.

Pour l'être humain, le diamètre de la cornée (avec limbe) est d'environ 12 à 13 mm avec une ora serrata d'environ 18 mm de diamètre.

A titre d'exemple on peut utiliser pour le traitement des adultes une électrode annulaire ou plusieurs électrodes en secteur d'anneau ayant un diamètre interne compris entre 12,5 et 14 mm et un diamètre externe compris entre 17 mm et 22 mm, ce qui correspond à une surface comprise entre 75 mm² et 250 mm², et de préférence compris entre 17 mm et 20 mm. Le courant peut être dans ce cas par exemple de 400µA et être appliqué pendant 4 minutes.

On remarquera que la disposition des électrodes actives, à savoir des électrodes surfaciques disposées en vis-à-vis de la ou des région(s) à traiter permet d'associer à un courant constant une densité de courant qui est elle-même constante et homogène sur toute la surface de la région à traiter.

Ceci présente plusieurs avantages.

En premier lieu, on évite que la densité de courant puisse atteindre localement des valeurs élevées dans certaines zones de la région à traiter et donc d'être à l'origine d'effets secondaires indésirables.

D'autre part, l'homogénéité de la densité de courant dans la région à traiter a pour effet que la pénétration du ou des produits actifs par exemple des médicaments est également homogène sur la région à traiter.

En aucun cas, l'électrode n'est en vis-à-vis de la rétine fonctionnelle.

Dans le cadre de la présente invention, l'administration d'au moins un produit actif par exemple un médicament s'effectue par l'intermédiaire des tissus qui permettent la meilleure pénétration du produit actif, dans le segment antérieur et postérieur : le limbe cornéoscléral, la conjonctive, la sclère, le corps ciliaire, la racine de l'iris, la parsplana, le vitré antérieur, la choroïde et la rétine non détachable non fonctionnelle.

L'absence de contact avec la cornée évite tout risque de lésion physique et chimique et en particulier des troubles oculaires transitoires ou permanents consécutifs au traitement, et elle permet également de dégager un espace central 23 permettant au praticien de contrôler le positionnement de l'appareil pendant tout le traitement.

De plus, on constate qu'à partir d'une certaine concentration de produit actif, qui varie en fonction de la nature du produit actif, le produit actif s'accumule dans certains tissus de l'oeil (espace sous-ténonien, sclère, espace suprachoroïdien et dans une moindre mesure iris I et corps ciliaire CC) avant d'être relargué progressivement vers d'autres tissus (choroïde CH, rétine RET), augmentant ainsi la durée d'action (temps de demi-vie avant l'élimination du produit actif).

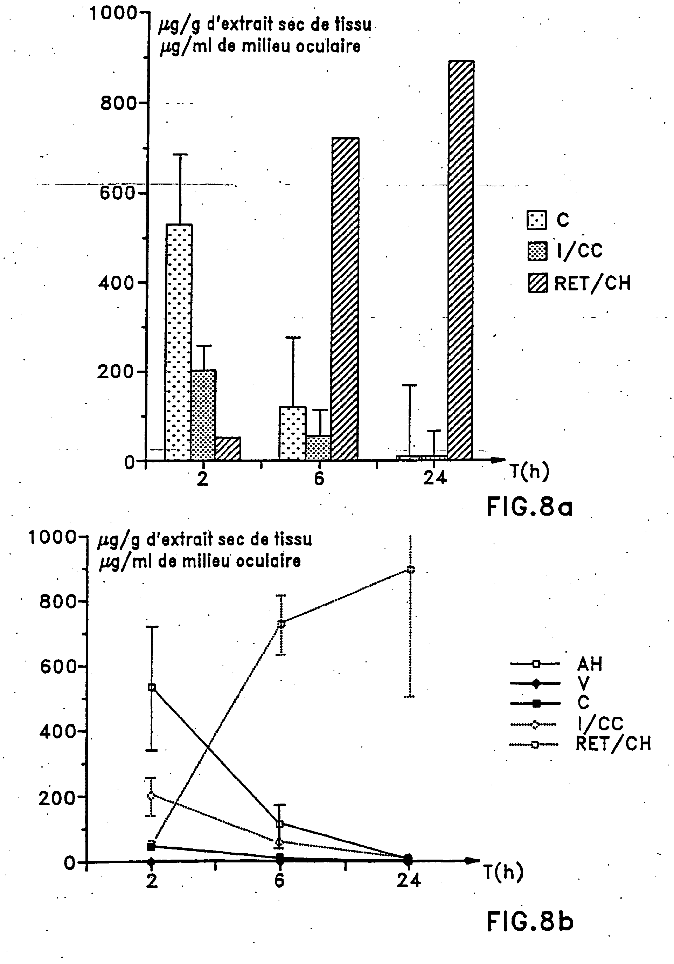

Ce phénomène est illustré par les courbes ci-jointes (Fig.8a et 8b), obtenues à partir d'expériences effectuées sur des lapins avec de l'hémisuccinate de méthylprednisolone (150 mg/ml, 2mA). Avec une solution à 62,5mg/ml, l'effet de relargage n'est pas observé. Le seuil de concentration permettant un relargage est d'environ 100mg/ml.

Le dispositif selon l'invention peut être de révolution, mais il est préférable qu'il soit sensiblement ovale pour tenir compte d'une part de la présence des paupières et d'autre part du profil légèrement ovale de la cornée.

Le dispositif représenté à la figure 7 présente une cavité ayant un profil externe elliptique de 20 mm d'axe focal parallèle à la ligne de fermeture des paupières, et de 18 mm de petit axe.

Un profil interne elliptique de la cavité de traitement peut présenter par exemple un grand axe parallèle à la ligne de fermeture des paupières et égal à 13,5 mm, et un petit axe perpendiculaire à cette ligne et égal à 12,5 mm.

Le dispositif représenté à la figure 7 présente quatre cavités 71 à 74 dont chacune présente une électrode active 75 à 78 alimentée par un circuit électronique individuel 79 à 82 analogue à celui représenté à la figure 1 et qui est intégré au dispositif. Les circuits électroniques sont alimentés par une pile 84 constituant le générateur de tension VS, et comportent une source de courant constant I régulée à une valeur choisie, et une temporisation T permettant de fixer le temps de traitement désiré. Alternativement, l'ensemble des circuits peut être disposé sur un circuit intégré unique, ou bien encore les fonctions peuvent être réparties sur plusieurs circuits internes reliés par un bus 85.

On notera que le réservoir peut être ovale ou bien présenter une forme allongée, par exemple elliptique.

Le réservoir et/ou l'électrode active peut être annulaire.

Il entre également dans le cadre de la présente invention que le réservoir présente un diamètre interne di avec D<di≤1,2D, D désignant le diamètre de la cornée, et un diamètre externe de, avec 1,4D<de≤1,8D, et de préférence 1,4D≤de≤1,7D.

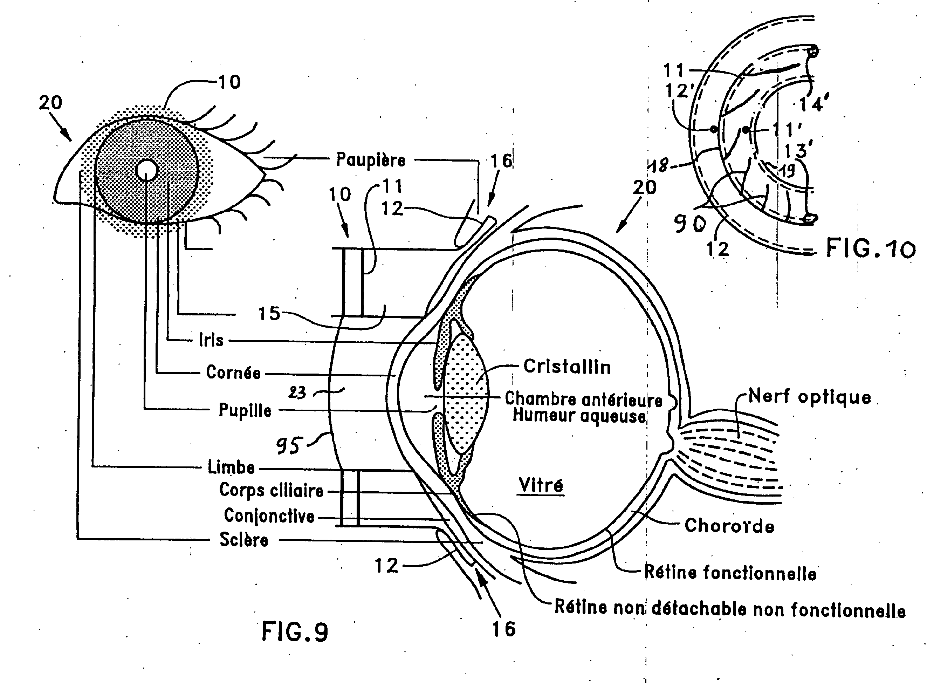

Le dispositif peut être maintenu en place à l'aide d'un dispositif de succion produisant une dépression comprise entre 35 mm Hg et 100 mm Hg et de préférence de l'ordre de 50 mm Hg. Cette dépression peut être en particulier générée à l'aide d'une membrane préférentiellement transparente 95 (Figure 9) qui obture la face externe de l'espace central 23, ce qui permet d'y créer une dépression par aspiration. Cette dépression peut être également créée par le praticien qui appuie sur la membrane 95 pour chasser de l'air de l'espace central, ce qui provoque après relâchement une dite dépression. La membrane 95 de mise en dépression étant transparente, le praticien peut contrôler le positionnement de l'appareil pendant le traitement, grâce à l'espace central 23.

Le produit actif peut être injecté par une seringue ou bien à partir d'un conteneur de produit actif adjacent au dispositif.

Lorsque le dispositif est en un matériau souple, ce qui est favorable à la mise en place et à l'étanchéité, les parois cylindriques externe 18 et interne 19 tendent à venir en contact l'une sur l'autre.

Pour y remédier, on dispose des ailettes 90, par exemple des ailettes planes radiales, et qui de préférence, s'étendent depuis une des parois cylindriques (18 ou 19) tout en restant espacées de l'autre paroi (19 ou 18) lorsque le dispositif est au repos. Pour faciliter l'évacuation des bulles d'air, on injecte la solution active dans le réservoir 15 par une entrée 13' (voir Figure 10) située à la partie inférieure ("position 6 heures") du dispositif posé sur l'oeil d'un patient dont la tête est inclinée vers l'arrière, et une ouverture 14' d'évacuation des bulles est prévue à la partie supérieure ("position 12 heures"). Pour aider l'évacuation des bulles, les ailettes 90, qui dans l'exemple représenté s'étendent à partir de la paroi 18, sont incurvées et sont convexes en direction de l'ouverture d'entrée 13'.

## Revendications

1. Dispositif de transfert d'au moins un produit actif dans le globe oculaire par iontophorèse, comprenant un réservoir annulaire (27,48,49) de produit actif susceptible d'être appliqué sur l'oeil d'un patient et une électrode passive (12), **caractérisé en ce que** le réservoir comporte une pluralité de compartiments en forme de secteurs annulaires (45,46,47) et **en ce que** le dispositif comporte en outre une pluralité d'électrodes actives surfaciques (41,42,43), chacune disposée dans un compartiment propre de sorte à être, en opération, en vis-à-vis d'au moins un tissu oculaire situé en périphérie de la cornée.

2. Dispositif selon la revendication 1, **caractérisé en ce que** chaque électrode active est alimentée par un circuit de contrôle individuel.

3. Dispositif selon l'une des revendications 1 à 2, **caractérisé en ce qu'**il comprend des moyens de remplissage (84,85,86,87) de compartiments en produits actifs, chaque moyen de remplissage étant propre à chaque compartiment, les moyens de remplissage comportant des ouvertures ménagées dans une paroi du réservoir, ainsi que des tubes qui s'étendent de l'intérieur à l'extérieur des compartiments.

4. Dispositif selon l'une des revendications précédentes, **caractérisé en ce qu'**il comprend dans chaque compartiment un produit actif différent de celui contenu dans chacun des autres compartiments, une fois le dispositif placé sur l'oeil et les compartiments remplis.

5. Dispositif selon la revendication précédente, **caractérisé en ce que** les différents produits actifs disposés dans les compartiments présentent un pH compris entre 6 et 8 et de préférence entre 7 et 7,6.

6. Dispositif selon l'une des revendications 1 à 4, **caractérisé en ce qu'**il comporte un dispositif de pompage permettant d'assurer une circulation des différentes solutions contenant les produits actifs dans les compartiments.

7. Dispositif selon la revendication 1, **caractérisé en ce que** le réservoir présente un diamètre interne di avec D<di≤1,2D, D désignant le diamètre de la cornée, et un diamètre externe de avec 1,4D<de≤1,8D, et de préférence 1,4D<de≤1,7D.

8. Dispositif selon la revendication 7, **caractérisé en ce que** le diamètre interne di est compris entre 12,5 mm et 14 mm et **en ce que** le diamètre externe de est compris entre 17 mm et 22 mm et de préférence entre 17 mm et 20 mm.

9. Dispositif selon l'une des revendications 1 à 6, **caractérisé en ce que** le réservoir a une forme allongée par exemple elliptique.

10. Dispositif selon l'une des revendications 1 à 6, **caractérisé en ce que** le dispositif est un ménisque plat.

11. Dispositif selon l'une des revendications précédentes, **caractérisé en ce qu'**il comporte en outre un générateur de courant constant (I) de densité nominale inférieure à 10mA/cm², et **en ce qu'**il comporte un dispositif de commande permettant d'appliquer ledit courant constant pendant une durée comprise entre 30 secondes et 10 minutes, et notamment entre 1 minute et 10 minutes.

12. Dispositif selon la revendication 11, **caractérisé en ce que** ladite densité de courant est comprise entre 0,1 mA/cm² et 5mA/cm².

13. Dispositif selon la revendication 12, **caractérisé en ce que** ladite densité de courant est comprise entre environ 0,2mA/cm² et environ 5,0Ma/cm², et notamment entre 0,8mA/cm² et 5,0mA/cm².

14. Dispositif selon l'une des revendications précédentes, **caractérisé en ce que** le réservoir est annulaire et présente des ailettes d'espacement entre sa paroi interne et sa paroi externe, ces ailettes étant radiales, ou bien incurvées de manière à être convexes vers une entrée de produits actifs.

## Claims

1. Device for transferring at least one active product into the eyeball by iontophoresis, comprising an annular reservoir (27, 48, 49) of active product, which can be applied to a patient's eye, and la passive electrode (12),
**characterized in that** the reservoir comprises a plurality of compartments in the form of annular sectors (45, 46, 47), and **in that** the device additionally comprises a plurality of active surface electrodes (41, 42, 43), each one arranged in its own compartment in such a way that, during operation, it lies opposite at least one ocular tissue situated at the periphery of the cornea.

2. Device according to Claim 1, **characterized in that** each active electrode is supplied by an individual control circuit.

3. Device according to one of Claims 1 and 2,
**characterized in that** it comprises filling means (84, 85, 86, 87) for filling compartments with active products, each filling means belonging to each compartment, the filling means comprising apertures, formed in a wall of the reservoir, and tubes that run from the inside to the outside of the compartments.

4. Device according to one of the preceding claims, **characterized in that** it comprises in each compartment an active product different from that contained in each of the other compartments, once the device has been placed on the eye and the compartments have been filled.

5. Device according to the preceding claim, **characterized in that** the different active products arranged in the compartments have a pH of between 6 and 8, preferably of between 7 and 7.6.

6. Device according to one of Claims 1 to 4,
**characterized in that** it comprises a pumping device ensuring circulation of the different solutions containing the active products in the compartments.

7. Device according to Claim 1, **characterized in that** the reservoir has an internal diameter di with D<di≤1.2 D, D denoting the diameter of the cornea, and an external diameter de with 1.4 D<de≤1.8 D, and preferably 1.4 D<de≤1.7 D.

8. Device according to Claim 7, **characterized in that** the internal diameter di lies between 12.5 mm and 14 mm, and **in that** the external diameter de lies between 17 mm and 22 mm, and preferably between 17 mm and 20 mm.

9. Device according to one of Claims 1 to 6,
**characterized in that** the reservoir has an elongate shape, for example an elliptical shape.

10. Device according to one of Claims 1 to 6,
**characterized in that** the device is a flat meniscus.

11. Device according to any of the preceding claims, **characterized in that** it additionally comprises a constant-current generator (I) with rated density less than 10 mA/cm², and **in that** it comprises a control device allowing said constant current to be applied for a period of time of between 30 seconds and 10 minutes, and in particular between 1 minute and 10 minutes.

12. Device according to Claim 11, **characterized in that** said current density lies between 0.1 mA/cm² and 5 mA/cm².

13. Device according to Claim 12, **characterized in that** said current density lies between about 0.2 mA/cm² and about 5.0 mA/cm², and in particular between 0.8 mA/cm² and 5.0 mA/cm².

14. Device according to one of the preceding claims, **characterized in that** the reservoir is annular and has spacing fins between its internal wall and its external wall, these fins being radial or, alternatively, curved so as to be convex towards an inlet for active products.

## Patentansprüche

1. Vorrichtung zur Übertragung wenigstens eines aktiven Mittels in den Augapfel durch lontophorese, die einen ringförmigen Behälter (27, 48, 49) des aktiven Wirkstoffs, der auf das Auge eines Patienten aufgesetzt werden kann, und eine passive Elektrode (12) umfasst, **dadurch gekennzeichnet, dass** der Behälter eine Vielzahl von Abteilungen in Form von Ringsegmenten (45, 46, 47) umfasst und **dadurch**, dass die Vorrichtung außerdem eine Vielzahl von aktiven Oberflächenelektroden (41, 42, 43) umfasst, von denen jede in einer eigenen Abteilung angeordnet ist, derart, dass sie bei der Anwendung gegenüber wenigstens einem Augengewebe liegt, das sich an der Peripherie der Hornhaut befindet.

2. Vorrichtung nach Anspruch 1, **dadurch gekennzeichnet, dass** jede aktive Elektrode von einer eigenen Steuerschaltung versorgt wird.

3. Vorrichtung nach einem der Ansprüche 1 bis 2, **dadurch gekennzeichnet, dass** sie Mittel zum Befüllen (84, 85, 86, 87) der Abteilungen mit einem aktiven Mittel umfasst, wobei jedes Befüllungsmittel zu einer Abteilung gehört und wobei die Befüllungsmittel Öffnungen umfassen, die in einer Wand des Behälters ausgebildet sind, sowie Röhren, die vom Inneren zum Äußeren der Abteilungen führen.

4. Vorrichtung nach einem der vorhergehenden Ansprüche, **dadurch gekennzeichnet, dass** sie, nachdem die Vorrichtung auf dem Auge platziert ist und die Abteilungen befüllt sind, in jeder Abteilung ein aktives Mittel enthält, das verschieden ist, von denjenigen in jeder der anderen Abteilungen.

5. Vorrichtung nach dem vorhergehenden Anspruch, **dadurch gekennzeichnet,**
**dass** die in die Abteilungen eingebrachten verschiedenen aktiven Mittel einen pH-Wert haben, der zwischen 6 und 8 und vorzugsweise zwischen 7 und 7,6 liegt.

6. Vorrichtung nach einem der Ansprüche 1 bis 4, **dadurch gekennzeichnet, dass** sie eine Pumpvorrichtung umfasst, die es erlaubt, in den Abteilungen eine Zirkulation verschiedener Lösungen, die die aktiven Mittel enthalten, sicherzustellen.

7. Vorrichtung nach Anspruch 1, **dadurch gekennzeichnet, dass** der Behälter einen Innendurchmesser di mit D < di≤ 1,2 D, wobei D den Durchmesser der Hornhaut bezeichnet, und einen Außendurchmesser de mit 1,4 < de ≤1,8 D, und vorzugsweise 1,4 D < de ≤ 1,7 D, aufweist.

8. Vorrichtung nach Anspruch 7, **dadurch gekennzeichnet, dass** der Innendurchmesser di zwischen 12,5 mm und 14 mm und dass der Außendurchmesser de zwischen 17 mm und 22 mm, und vorzugsweise zwischen 17 mm und 20 mm, liegt.

9. Vorrichtung nach einem der Ansprüche 1 bis 6, **dadurch gekennzeichnet, dass** der Behälter eine längliche, beispielsweise elliptische Form hat.

10. Vorrichtung nach einem der Ansprüche 1 bis 6, **dadurch gekennzeichnet, dass** die Vorrichtung ein flacher Meniskus ist.

11. Vorrichtung nach einem der vorhergehenden Ansprüche, **dadurch gekennzeichnet, dass** sie außerdem einen Konstantstromerzeuger umfasst, mit einem konstanten Strom (I) mit einer Nenndichte kleiner als 10 mA/cm², und dass sie eine Steuervorrichtung umfasst, die es erlaubt, den Konstantstrom während einer Dauer anzuwenden, die zwischen 30 Sekunden und 10 Minuten, und insbesondere zwischen 1 Minute und 10 Minuten liegt.

12. Vorrichtung nach Anspruch 11, **dadurch gekennzeichnet, dass** die Stromdichte zwischen 0,1 mA/cm² und 5 mA/cm² liegt.

13. Vorrichtung nach Anspruch 12, **dadurch gekennzeichnet, dass** die Stromdichte zwischen etwa 0,2 mA/cm² und etwa 5,0 mA/cm² und insbesondere zwischen 0,8 mA/cm² und etwa 5,0 mA/cm² liegt.

14. Vorrichtung nach einem der vorhergehenden Ansprüche, **dadurch gekennzeichnet, dass** der Behälter ringförmig ist und zwischen seiner Innenwand und seiner Außenwand Abstandsflügel aufweist, wobei diese Flügel radial sind oder auch derart gebogen, dass sie in Richtung eines Eintritts des aktiven Wirkstoffs konvex sind.
